(19) Europäisches Patentamt
European Patent Office
Office européen des brevets

(11) Veröffentlichungsnummer: **0 162 235**
**B1**

# (12) EUROPÄISCHE PATENTSCHRIFT

(45) Veröffentlichungstag der Patentschrift: **18.01.89**

(51) Int. Cl.[4]: **C 07 D 407/12**, C 07 D 307/93, C 07 D 493/08 // (C07D493/08, 317:00, 311:00)

(21) Anmeldenummer: **85103713.5**

(22) Anmeldetag: **29.03.85**

## (54) Enantiomerenreine acetalisch mono-geschützte Diole, deren Herstellung und Verwendung.

(30) Priorität: **04.04.84 AT 1136/84**

(43) Veröffentlichungstag der Anmeldung:
**27.11.85 Patentblatt 85/48**

(45) Bekanntmachung des Hinweises auf die Patenterteilung:
**18.01.89 Patentblatt 89/3**

(84) Benannte Vertragsstaaten:
**AT BE CH DE FR GB IT LI LU NL SE**

(56) Entgegenhaltungen:
**EP-A- 0 083 335**

**TETRAHEDRON LETTERS, Band 22, Nr. 40, 1981, Seiten 4021-4024, Pergamon Press Ltd., Oxford, GB; R. BERNARDI u.a.: "On the steric course of addition of Grignard reagents onto alpha,beta-dialkoxy Erythro and Threo chiral aldehydes. Synthesis of (+) and (-)-Exo and Endo-Brevicomin"**

(73) Patentinhaber: **AGROLINZ AGRARCHEMIKALIEN GESELLSCHAFT M.B.H., St. Peterstrasse 25, A-4021 Linz (AT)**

(84) Benannte Vertragsstaaten: **BE CH FR GB IT LI LU NL SE AT**

(73) Patentinhaber: **Lentia Gesellschaft mit beschränkter Haftung, Arabellastrasse 4 Postfach 81 05 08, D-8000 München 81 (DE)**

(84) Benannte Vertragsstaaten: **DE**

(72) Erfinder: **Noe, Christian Roland, Dr., Stösslgasse 9, A-1130 Wien (AT)**
Erfinder: **Knollmüller, Max, Dr., Schrottgasse 11/20, A-1030 Wien (AT)**
Erfinder: **Kürner, Helmut Hermann, Dipl.-Ing., Brunnengasse 205, A-2632 Grafenbach St. Valentin (AT)**

(74) Vertreter: **Kunz, Ekkehard, Dr., Chemie Holding AG Patentwesen St. Peter-Strasse 25, A-4021 Linz (AT)**

**EP 0 162 235 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Anmeldung betrifft enantiomerenreine acetalisch mono-geschützte Diole, deren Herstellung und Verwendung zur Herstellung der Pheromone (1R-exo)-Brevicomin, (1S-exo)-Brevicomin, (1R-endo)-Brevicomin und (1S-endo)-Brevicomin.

Pheromone gewinnen wegen ihrer Wirkungsspezifität und Umweltfreundlichkeit zunehmend an Bedeutung im Pflanzenschutz. Besondere Beachtung haben unter anderem die Pheromone von Kieferborkenkäfern, nämlich exo-Brevicomin und endo-Brevicomin, gefunden. Während das Pheromon exo-Brevicomin als Lockstoff wirkt, weist endo-Brevicomin eine Repellentwirkung auf. Die Brevicomine sind chirale Strukturen. Das bedeutet, dass es zum Auftreten von optischen Antipoden (= Enantiomeren) kommt. Wie für physiologisch aktive Verbindungen durchaus zu erwarten, zeigt im Falle des exo-Brevicomins nur 1 Enantiomer, nämlich das (1R-exo)-Brevicomin, die gewünschte anlockende Wirkung, während das andere inaktiv ist. Für endo-Brevicomin liegen entsprechende Untersuchungen bisher nicht vor.

Brevicomin leitet sich von der Verbindung 6,7-Dihydroxy-2-nonanon ab, aus der es durch Säurekatalyse unter Cyclisierung erhalten wird. Da das 6,7-Dihydroxy-2-nonanon 2 Chiralitätszentren aufweist, können 4 verschiedene Brevicomine entstehen.

Es sind dies:

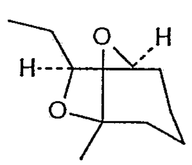

(1S-exo)-Brevicomin

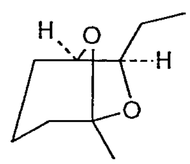

(1R-exo)-Brevicomin

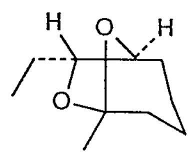

(1S-endo)-Brevicomin

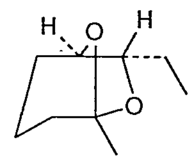

(1R-endo)-Brevicomin

Um bei der Cyclisierung von 6,7-Dihydroxy-2-nonanon nur eines dieser Brevicomine zu erhalten, ist eine definierte räumliche Anordnung jeder der beiden Hydroxylgruppen Voraussetzung. Dazu ist der gezielte Einsatz von Schutzgruppen notwendig. Ein geschütztes enantiomeren- und diastereomerenreines Dihydroxynonanon mit einer Konfiguration der Hydroxylgruppen, welches bei der Schutzgruppenabspaltung zu einem der beiden enantiomerenreinen endo-Brevicomine führt, ist nicht bekannt. Geschützte Nonanone, die zu einem der exo-Brevicomine führen, sind wohl beschrieben, jedoch synthetisch schwer zugänglich, wobei meist auch die Schutzgruppenabspaltung schwieriger ist.

Ein Gemisch aus exo- und endo-Brevicominen kann in einer nicht stereoselektiven Synthese nach Bernardi, Tetrahedron Letters, Vol. 22, No. 40, pp. 4021–4024 (1981) erhalten werden.

Nach Meyer, Liebigs Ann. Chem. 1977, 732–736, wird nach einer vielstufigen Synthese, ausgehend von natürlicher Weinsäure, das biologisch inaktive (l-S-exo)-Brevicomin erhalten. Verbindungen, aus denen die endo-Brevicomine erhalten werden, sind nicht angegeben.

Nach Ferrier, J. Chem. Soc. Perkin Trans I, 1645–1647 (1983), wird (+)exo-Brevicomin aus einem Dihydroxynonanon erhalten, indem beide Hydroxylgruppen durch Benzoylgruppen geschützt sind, wobei jedoch die Schutzgruppenabspaltung und Cyclisierung in 2 Stufen, alkalisch und sauer, erfolgen muss. Das geschützte Dihydroxynonanon ist nur schwer aus einem selektiv geschützten Kohlenhydratderivat zugänglich.

Demgegenüber konnte nun ein Verbindungstyp eines geschützten Dihydroxynonanons gefunden werden, der in allen vier möglichen Konfigurationen der beiden Hydroxylgruppen leicht zugänglich ist. Da in diesem Verbindungstyp sowohl die beiden endo-Brevicomine als auch die beiden exo-Brevicomine selektiv in ihrer Konfiguration vorgebildet sind, entsteht bei Säurebehandlung durch Schutzgruppenabspaltung und gleichzeitige spontane Cyclisierung aus dem jeweils vorgebildeten geschützten Dihydroxynonanon genau das entsprechende enantiomerenreine Brevicomin.

Gegenstand der vorliegenden Erfindung sind demnach enantiomerenreine acetalisch monogeschützte Diole der allgemeinen Formel

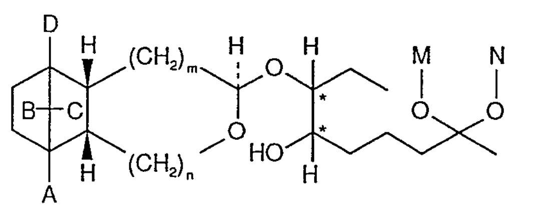

I,

in welcher A, B, C und D Wasserstoff oder eine Methylgruppe in beliebiger Kombination, M und N Keton-Schutzgruppen vom Ketal-Typ, m und n die Zahlen 0, 1 oder 2 bedeuten, wobei die Summe aus m und n die Zahl 1 oder 2 sein muss.

Bevorzugte Verbindungen der allgemeinen Formel I sind solche, in denen das Bicycloheptan-Ringsystem ein Bornanringsystem (A, B, C = Methyl, D = H) ist, da solche Verbindungen sowohl als (R)- als auch als (S)-Enantiomere relativ leicht aus gängigen Naturstoffen (D-Campher, L-Borneol) zugänglich sind. Die bevorzugte Bedeutung für n ist die Zahl 0. Die Bedeutung der Ketonschutzgruppen vom Ketal-Typ M und N ist weniger gross, sie bedeuten beispielsweise aliphatische oder araliphatische Kohlenwasserstoffe, bevorzugt bedeuten sie je eine Alkylgruppe mit 1 bis 5 Kohlenstoffatomen oder gemeinsam eine Alkylengruppe mit 2 bis 7 Kohlenstoffatomen, etwa eine Äthylen- oder Propylengruppe.

Ein weiterer Gegenstand der Erfindung ist ein Verfahren zur Herstellung der enantiomerenreinen acetalisch mono-geschützten Diole der allgemeinen Formel I sowie deren Verwendung zur Herstellung enantiomerenreiner Brevicomine.

Die tricyclische acetalische Schutzgruppe ist in EPA-008335 beschrieben, zur Herstellung der Ausgangsverbindung der allgemeinen Formel II des Formelblattes, in der A, B, C, D, m und n die in Formel I angegebene Bedeutung haben, wird racemisches 2-Hydroxybutannitril zunächst mit einem in EP-A 008 335 beschriebenen, enantiomerenreinen anomerselektiven Lactol oder einer seiner Anhydroformen umgesetzt und die erhaltenen Diastereomeren getrennt.

Die Herstellung der Verbindungen der allgemeinen Formel I erfolgt, indem das enantiomerenreine Diastereomer der allgemeinen Formel II mit einer Grignard-Verbindung der allgemeinen Formel III des Formelblattes, in der X Chlor oder Brom bedeutet, in Gegenwart eines für Grignard-Reaktionen geeigneten Lösungsmittels, wie Diethylether oder Tetrahydrofuran, etwa bei Temperaturen von −20 bis +30°C zum enantiomerenreinen Keton der allgemeinen Formel IV des Formelblattes, in der A, B, C, D, m, n, M und N die in Formel I angegebene Bedeutung haben, umgesetzt wird. Beim nächsten Schritt, der Reduktion der Ketogruppe zur Hydroxygruppe (allgemeine Formel I), erfolgt die Bildung eines neuen Asymmetriezentrums. Durch Wahl der Reduktionsbedingungen lassen sich beträchtliche Selektivitäten erzielen, nach denen die beiden Wasserstoffatome in den nunmehr vorhandenen beiden Asymmetriezentren zueinander entweder in cis-Anordnung oder in trans-Anordnung stehen.

Erfolgt die Reduktion durch komplexe Hydride, wie Lithiumaluminiumhydrid, Natrium-bis-(2-methoxyethoxy)-aluminiumhydrid oder insbesondere Zinkborhydrid, so stehen nach der Reduktion die beiden Wasserstoffatome zueinander bevorzugt in cis-Anordnung, nach Schutzgruppenabspaltung und Cyclisierung entsteht endo-Brevicomin. Die Reduktion mit komplexen Hydriden kann etwa in Äthern oder aromatischen Kohlenwasserstoffen, wie Toluol, durchgeführt werden. Besonders bevorzugt sind nichtcyclische Äther, wie Diethylether. Die Reduktion kann etwa bei Temperaturen von −100 bis +40°C durchgeführt werden.

Erfolgt die Reduktion durch nicht komplexe Hydride, wie Boran- oder Alan-Verbindungen, wie Diisobutylaluminumhydrid (Diba H), so stehen nach der Reduktion die beiden Wasserstoffatome zueinander bevorzugt in trans-Anordnung, nach Schutzgruppenabspaltung und Cyclisierung entsteht exo-Brevicomin. Die Wahl des Lösungsmittels hat bei der Reduktion mit nicht komplexen Hydriden weniger Einfluss, geeignete Lösungsmittel sind etwa aromatische oder aliphatische Kohlenwasserstoffe, wie Toluol, Hexan, oder cyclische oder nichtcyclische Äther, wie Tetrahydrofuran oder Diethylether. Die Reduktion kann etwa bei Temperaturen von −100 bis +40°C durchgeführt werden.

Bei beiden Reduktionsarten kann gegebenenfalls das als Nebenprodukt gebildete Diastereomere abgetrennt werden. Von besonderem Vorteil ist hierbei die gute Trennbarkeit, die auf die enantiomerenreine tricyclische acetalische Schutzgruppe zurückgeführt werden kann. Die Trennung erfolgt vorzugsweise durch Chromatographie, doch ist bei kristallisierten Verbindungen auch eine Trennung durch Kristallisation möglich.

Die Abspaltung sämtlicher Schutzgruppen erfolgt in einem Schritt durch Zugabe katalytischer Mengen einer zur Acetalspaltung geeigneten Säure, z.B. Salzsäure, Toluolsulfonsäure, Schwefelsäure, oder von stark sauren Ionenaustauschern, wobei sogleich spontane Cyclisierung erfolgt. Die Schutzgruppenabspaltung erfolgt vorzugsweise in Gegenwart einer Mercaptosäure, welche bevorzugt mit der acetalischen Schutzgruppe reagiert. Dadurch wird eine einfache Reinigung des Brevicomins durch Extraktion des als Nebenprodukt entstandenen Thioacetals in die alkalische wässrige Phase ermöglicht. Aus diesem Thioacetal kann das als Schutzgruppe verwendete tricyclische Ringsystem leicht rückgewonnen werden, indem es etwa mit Methanol zum entsprechenden Methylacetal umgesetzt wird, das mit racemischen 2-Hydroxybutannitril wieder die Ausgangsverbindung der allgemeinen Formel II ergibt.

Beispiel 1:
[2R-(2α(R*),3aα,4α,7d,7aα)]-1-(2-Methyl-1,3-dioxolan-2-yl)-5-[(octahydro-7,8,8-trimethyl-4,7-methanobenzofuran-2-yl)-oxy]-heptan-4-on (Verbindung der Formel IV (R))

0,9 g[2R-(2α(R*),3aα,4α,7α,7aα)]-2-[(octahydro-7,8,8-trimethyl-4,7-methanobenzofuran-2-yl)-oxy]-butannitril (Verbindung der Formel II(R)) in 10 ml wasserfreiem Ether werden mit 7 ml 1molarer etherischer Grignardlösung von 2-(3-Chlorpropyl)-2-methyl-1,3-dioxolan (Verbindung der Formel III, X = Cl) versetzt. Es wird auf 0°C erwärmen gelassen und 3 Stunden bei dieser Temperatur gerührt. Es wird mit 10 ml Petrolether verdünnt und auf 20 ml 10%ige Essigsäure gegossen. Die wässrige Phase wird mit Petrolether extrahiert, die vereinigten organischen Phasen mit Natriumhydrogencarbonat-Lösung gewaschen, getrocknet, eingedampft und der Rückstand über 50 g Triethylamin-imprägniertes Kieselgel (Korngrösse 0,04–0,063) chromatographiert. Es werden 0,93 g (69% der Theorie) obiger Verbindung der Formel IV erhalten. Farbloses Öl, Kp. 100°C/0,005 mm (Luftbad); $\alpha_D^{20}$ = +105,6° (c = 1,40 in Dichlormethan); $^1$H-NMR ($CDCl_3$):δ = 5,25 (dd, 1H) 4,24 (dd, $J_1$ = 9,5 Hz, $J_2$ = 1 Hz, 1H), 4,03 (dd; 1H), 3,92 (s; 4H), 3,2–2,77 (m; 1H), 2,6–2,4 (m; 2H), 2,0–1,2 (m; 13H), 1,31 (s; 3H), 0,92 (t; 3H), 0,98/0,92/0,87 (3s; 9H).

Die Ausgangsprodukte der allgemeinen Formel II können folgenderweise hergestellt werden.

Eine Lösung von 10 g rac. 2-Hydroxybutannitril und 19,6 g (0,1 mol) acetalische Schutzgruppe in 300 ml Dichlormethan wird nach Zugabe von 0,5 g p-Toluolsulfonsäure 20 Stunden bei Raumtemperatur stehengelassen. Es wird mit Natriumhydrogencarbonatlösung gewaschen, die organische Phase getrocknet, eingedampft und der Rückstand über 660 g Triethylamin-imprägniertes Kieselgel (Korngrösse 0,04–0,063) chromatographisch aufgetrennt (Eluens: Petrolether/Ether = 15:1). Es werden 11,5 g [2R-(2α(R*),3aα,4α,7α,7aα)]-2-[(octahydro-7,8,8-trimethyl-4,7-methanobenzofuran-2-yl)-oxy]-butannitril (Verbindung der Formel II(R)) erhalten.

Anschliessend werden 8 g [2R-(2α(S*)-3aα,4α,7α,7aα)]-2-[(octahydro-7,8,8-trimethyl-4,7-methanobenzofuran-2-yl)oxy]-butannitril (Verbindung der Formel II (S)) erhalten. Eluens: Petrolether/Ether = 8:1.

Verbindung der Formel II (R):

Farbloses Öl, Kp. 80°C/0,005 mm (Luftbad); $[\alpha]_D^{20}$ = +201° (c = 1,66 in Dichlormethan); $^1$H-NMR ($CDCl_3$): δ = 5,52 (d, J = 4,4 Hz; 1H), 4,39 (t, J = 6,5 Hz, 1H), 4,17 (d, J = 9,3 Hz; 1H), 2,7–3,1 (m; 1H), 2,0–1,1 (m; 9H), 1,05 (t, J = 7,4 Hz; 3H), 0,96/0,92/0,87 (3s; 9H).

Verbindung der Formel II (S):

Farblose Kristalle, Fp. 56–57°C; $[\alpha]_D^{20}$ = +95,3° (c = 0,51 in Dichlormethan); $^1$H-NMR ($CDCl_3$): δ = 5,38 (d, J = 4 Hz; 1H), 4,45 (d, J = 9,5 Hz; 1H), 4,14 (t, J = 6,5 Hz; 1H), 3,2–2,7 (m; 1H), 1,9–1,2 (m; 9H), 1,05 (t, J = 7 Hz; 3H), 1,01/0,93/0,90 (3s; 9H).

Beispiel 2:

[2R-(2α(S*),3aα,4α,7α,7aα)]-1-(2-Methyl-1,3-dioxolan-2-yl)-5-[(octahydro-7,8,8-trimethyl-4,7-methanobenzofuran-2-yl)-oxy]-heptan-4-on (Verbindung der Formel IV (S))

1 g der Verbindung der Formel II(S), hergestellt nach Beispiel 1, in 10 ml wasserfreiem Ether wird mit 7,5 ml 1molarer etherischer Grignard-Lösung von 2-(3-Chlorpropyl)-2-methyl-1,3-dioxolan (Verbindung der Formel III, X = Cl) versetzt. Es wird auf 0°C erwärmen gelassen und 3 Stunden bei dieser Temperatur gerührt. Es wird mit 10 ml Petrolether verdünnt, 20 ml 10%ige Essigsäure werden zugesetzt und weitere 10 Minuten gerührt. Es wird mit Petrolether extrahiert, die vereinigten organischen Phasen mit gesättigter Natriumbicarbonatlösung gewaschen, getrocknet ($Na_2SO_4$) und im Vakuum eingedampft. Der Rückstand wird durch Säulenchromatographie über 100 g Triethylamin-imprägniertes Kieselgel gereinigt. Es werden 1,32 g obiger Verbindung der Formel IV(S) erhalten (88%), farbloses Öl, Kp. 110°C/0,01 mm (Luftbad); $[\alpha]_D^{20}$ = +57,0° (c = 2,81 in Dichlormethan); $^1$H-NMR ($CDCl_3$): δ = 5,30 (dd, 1H), 4,17 (d, J = 11 Hz; 1H), 3,92 (s; 4H), 3,81 (t, J = 6,5 Hz; 1H), 3,2–2,8 (m; 1H), 2,6–2,45 (m; 2H), 2,0–1,1 (m; 16H, darin: 1,31: s; 3H), 0,92 (t; 3H), 0,98/0,90/0,85 (3s; 9H)

Beispiel 3: Reduktion mit DibalH

Es werden in eine Lösung von 2,9 g [2R-(2α(R*)-3aα,4α,7α,7aα)]-1-(2-Methyl-1,3-dioxolan-2-yl)-5-[(octahydro-7,8,8-trimethyl-4,7-methanobenzofuran-2-yl)-oxy]-heptan-4-on (Verbindung der Formel IV(R)) in 50 ml absol. Toluol bei −60°C unter $N_2$ 10 ml einer 1,2molaren Lösung von DibalH in n-Hexan zugetropft. Nach 45 Minuten bei −60°C wird 1N NaOH zugegeben und das Reaktionsgemisch zwischen Ether und verdünnter NaOH verteilt, die organische Phase getrocknet und eingedampft. Der Rückstand wird über 350 g Triethylamin-imprägniertes Kieselgel (Korngrösse 0,04–0,063) mit Petrolether/Ether = 1:1 als Eluens aufgetrennt. Es werden 800 mg [2R-(2α(R*,R*)-3aα,4α,7d,7aα)]-1-(2-Methyl-1,3-dioxolan-2-yl)-5-[(octahydro-7,8,8-trimethyl-4,7-methanobenzofuran-2-yl)-oxy]-heptan-4-ol (Verbindung der Formel I (4R, 5R)) und 135 mg [2R-(2α(R*,S*), 3aα,4α,7α,7aα)]-1-(2-Methyl-1,3-dioxolan-2-yl)-5-[(octahydro-7,8,8-trimethyl-4,7-methanobenzofuran-2-yl)-oxy]-heptan-4-ol (Verbindung der Formel I(4S,5R)) als Reinfraktionen erhalten. Durch weitere säulenchromatographische Auftrennung der Gemischfraktionen können noch 920 mg der Verbindung der Formel I(4R,5R) und 550 mg der Verbindung der Formel I(4S,5R) rein isoliert werden.

Verbindung der Formel I(4R,5R):

Farbloses Öl, Kp. 130°C/0,003 mm (Luftbad); $[\alpha]_D^{20}$ = +82,8° (c = 0,58 in Dichlormethan); $^1$H-NMR ($CDCl_3$): δ = 5,43 (d, J = 2,9 Hz; 1H), 4,31 (d, J = 9,5 Hz; 1H), 3,93 (s; 4H), 3,7–3,2 (m; 2H), 3,2–2,7 (m; 1H), 2,15–1,1 (m; 18H, darin: 1,3: s; 3H), 1,0 (t; 3H), 0,98/0,92/0,87 (3s; 9H).

Verbindung der Formel I(4S,5R):

Farbloses Öl, Kp. 130°C/0,003 mm (Luftbad); $[\alpha]_D^{20}$ = +85,2° (c = 2,68 in Dichlormethan); $^1$H-NMR ($CCl_4$): δ = 5,48 (d, J = 4,5 Hz; 1H), 4,29 (d, J = 10 Hz; 1H), 3,92 (s; 4H), 3,9–3,45 (m; 2H), 3,2–2,8 (m; 1H), 2,1–1,1 (m; 18H, darin 1,28: s; 3H), 0,9 (t; 3H), 0,96/0,90/0,85 (3s; 9H).

Herstellung von (1R-exo)-Brevicomin:

330 mg obiger Verbindung der Formel I(4R,5R) und 352 mg 2-Mercaptopropansäure werden in 10 ml Dichlormethan gelöst und nach Zugabe katalytischer Mengen von p-Toluolsulfonsäure 1 Stunde bei Raumtemperatur gerührt. Es wird mit 1N NaOH extrahiert, die organische Phase getrocknet und das Lösungsmittel über eine Kolonne abdestilliert. Das Rohprodukt wird im Kugelrohr destilliert. Es werden 94 mg (1R-exo)-Brevicomin erhalten, farbloses Öl; $[\alpha]_D^{20}$ = +83° (in Ether); $^1$H-NMR ($CDCl_3$): δ = 4,0 (breit; 1H), 3,8 (t, 6,5 Hz; 1H), 1,9–1,4 (m; 8H), 1,3 (s; 3H), 0,88 (t, 6,8 Hz; 3H).

Herstellung von (1S-endo)-Brevicomin:

1,7 g obiger Verbindung der Formel I (4S,5R) und 1,4 g 2-Mercaptopropansäure in 30 ml Dichlormethan werden nach Zusatz von katalytischen Mengen p-Toluolsulfonsäure 1 Stunde bei Raumtemperatur stehengelassen. Anschliessend wird mit 1N NaOH extrahiert, die organische Phase getrocknet und das Lösungsmittel über eine Kolonne abdestilliert. Das Rohprodukt wird im

Kugelrohr destilliert. Es werden 540 mg (1S-endo)-Brevicomin (S-II) erhalten, farbloses Öl; $[\alpha]_D^{20}$ = −71° (in Ether); $^1$H-NMR ($CCl_4$: δ = 4,03 (breit; 1H), 3,8 (m; 1H), 1,9–1,4(m; 8H), 1,3 (s; 3H), 0,95 (t; 3H).

Beispiel 4: Reduktion mit Boran in Thiobismethan

Eine Lösung von 150 mg der Verbindung der Formel IV(R), hergestellt nach Beispiel 1, in 5 ml wasserfreiem Ether wird 1 mmol Boran in Thiobismethan bei −55°C unter $N_2$ versetzt. Nach 1 Stunde bei −55°C wird die Lösung mit 10 ml 1N NaOH hydrolisiert, mit Ether extrahiert, die organische Phase mit Wasser geschüttelt, getrocknet und eingedampft. Es werden 140 mg (93%) einer Mischung der Verbindungen der Formel I(4R,5R): I(4R,5S) = 3 : 1 erhalten, aus welcher nach dem im Beispiel 3 angegebenen Trennverfahren 95 mg der Verbindung der Formel I(4R,5R) und 32 mg der Verbindung der Formel I(4S,5R) erhalten werden können.

Beispiel 5: Reduktion mit Lithiumaluminiumhydrid

Zu einer Suspension von 12 mg Lithiumaluminiumhydrid in 5 ml wasserfreiem Ether werden bei 0°C unter $N_2$ 210 mg der Verbindung der Formel IV(R), hergestellt nach Beispiel 1, gelöst in 20 ml wasserfreiem Ether zugetropft. Nach 15 Minuten bei 0°C wird mit Wasser hydrolisiert, der entstandene Niederschlag über Hyflo abgenutscht und mit Ether sorgfältig nachgewaschen. Die organische Phase wird mit Wasser gewaschen, getrocknet und eingedampft. Es werden 205 mg einer Mischung der Verbindung der Formel I(4R,5R) und I(4S,5R) mit einem Verhältnis von 1:2 erhalten, aus welchem nach dem in Beispiel 3 gegebenen Trennverfahren 61 mg der Verbindung der Formel I(4R,5R) und 122 mg der Verbindung der Formel I(4S,5R) erhalten werden.

Beispiel 6: Reduktion mit Natrium-bis-(2-methoxyethoxy)aluminiumhydrid

In eine Lösung von 205 mg der Verbindung der Formel IV(R), hergestellt nach Beispiel 1, in 5 ml wasserfreiem Ether werden unter $N_2$ bei −20°C 1,2 mmol Natrium-bis-(2-methoxyethoxy)-aluminiumhydrid (Redal) eingespritzt. Nach 30 Minuten wird mit 1N NaOH hydrolysiert, mit Ether extrahiert, die organische Phase getrocknet und eingedampft. Es werden 200 mg (97%) einer Mischung der Verbindungen der Formel I(4R,5R) und (4S,5R) erhalten, aus welchem nach dem in Beispiel 3 gegebenen Trennverfahren 51 mg der Verbindung der Formel I(4R,5R) und 129 mg der Verbindung der Formel I(4S,5R) erhalten werden.

Beispiel 7: Reduktion mit Zinkborhydrid

Eine Lösung von 220 mg der Verbindung der Formel IV(R), hergestellt nach Beispiel 1, in 7 ml wasserfreiem Ether wird mit 6 ml 0,2 molaren Zinkborhydrid in Ether bei -40°C unter $N_2$ versetzt. Nach 1 Stunde wird mit 1N NaOH hydrolisiert, mit Ether extrahiert, die organische Phasen mit Wasser geschüttelt, getrocknet und eingedampft. Es werden 215 mg einer Mischung der Verbindungen der Formel I(4R,5R) und I(4S,5R) im Verhältnis 1 : 19 erhalten, aus welchem nach dem in Beispiel 3 angegebenen Trennverfahren 190 mg der Verbindung der Formel I(4S,5R) erhalten werden.

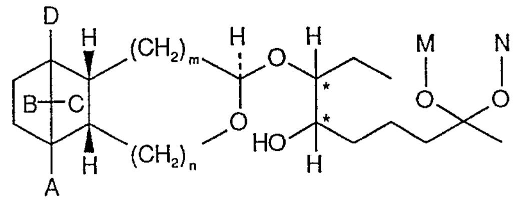

I,

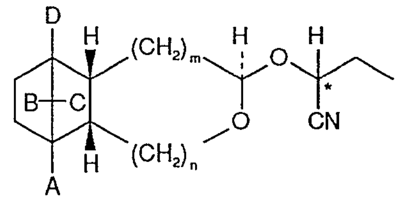

II,

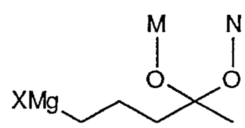

III,

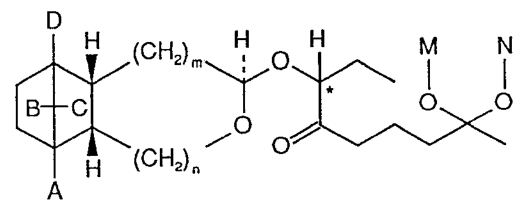

IV,

Beispiel 8: Reduktion mit Zinkborhydrid

Eine Lösung von 150 mg der Verbindung der Formel IV(S), hergestellt nach Beispiel 2, in 5 ml wasserfreiem Ether wird mit 4 ml 0,2 molarer Zinkborhydridlösung in Ether bei 0°C unter $N_2$ versetzt. Nach 0,5 Stunden wird mit 1N NaOH hydrolisiert, mit Ether extrahiert, die organische Phase mit Wasser geschüttelt, getrocknet und eingedampft. Es werden 145 mg einer Mischung der Verbindungen der Formel I(4S,5S) und I(4R,5S) im Verhältnis 1 : 19 erhalten, aus welchem nach dem in Beispiel 3 angegebenen Trennverfahren die reine Verbindung der Formel I(4R,5S) erhalten wird.

[2R-(2α(4R*,5S*)-3aα,4α,7α,7aα)]-1-(2-Methyl-1,3-dioxolan-2-yl)-5-[(oxtahydro-7,8,8-trimethyl-4,7-methanobenzofuran-2-yl)-oxy]-heptan-4-ol: farbloses Öl, Kp. 130°C/0,003 mm (Luftbad); $^1$H-NMR ($CDCl_3$):δ = 5,33 (dd; 1H), 4,34 (dd, $J_1$ = 1 Hz, $J_2$ = 8,5 Hz; 1H), 3,92 (s; 1H) 3,8–3,2 (m; 3H), 3,2–2,7 (m; 1H), 2,1–1,0 (m; 15H), 1,32 (s; 3H), 1,0–0,85 (3H), 0,97/0,92/0,86 (s; 9H).

Beispiel 9: Reduktion mit DibalH

Es werden in eine Lösung von 185 mg der Verbindung der Formel IV(S), hergestellt nach Beispiel 2, in 7 ml wasserfreiem Ether bei −60°C unter $N_2$0,5 ml 1,2 molare DibalH-Lösung in n-Hexan gespritzt. Nach 20 Minuten bei −60°C wird 1N NaOH zugegeben, das Reaktionsgemisch zwischen Ether und verdünner NaOH verteilt, die organische Phase getrocknet und eingedampft. Es werden 180 mg (97%) einer Mischung der Verbindungen der Formel I(4S,5S) und I(4R,5S) im Verhältnis 2:1 erhalten. Die Verbindung der Formel I(4S,5S) wird in reiner Form durch Säulenchormatographie erhalten.

[2R-(2α(4S*,5S*)-3aα,4α,7α,7aα)]-1-(2-Methyl-1,3-dioxolan-2-yl)-5-[(octahydro-7,8,8-trimethyl-4,7-methanobenzofuran-2-yl)-oxy]-heptan-4-ol: farbloses Öl, Kp. 130°C/0,003 mm (Luftbad); $^{1}$H-NMR ($CDCl_3$): δ = 5,36 (dd, 1H), 4,36 (d, J = 9,3 Hz; 1H), 3,92 (s; 4H), 3,9–2,6(m; 3H), 2,15–1,10 (m; 16H), 1,32 (s; 3H), 1,0–0,85 (3H), 0,95/0,91/0,86 (s; 9H).

**Patentansprüche für die Vertragsstaaten BE, CH, DE, FR, GB, IT, LI, LU, NL, SE**

1. Enantiomerenreine acetalisch mono-geschützte Diole der allgemeinen Formel

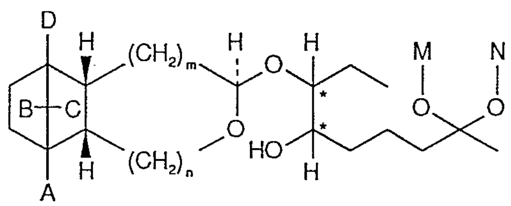

I,

in welcher A, B, C und D Wasserstoff oder eine Methylgruppe in beliebiger Kombination, M und N Keton-Schutzgruppen vom Ketal-Typ, m und n die Zahlen 0, 1 oder 2 bedeuten, wobei die Summe aus m und n die Zahl 1 oder 2 sein muss.

2. Enantiomerenreine acetalisch mono-geschützte Diole der allgemeinen Formel I gemäss Anspruch 1, dadurch gekennzeichnet, dass A, B und C je eine Methylgruppe, D Wasserstoff, M und N je eine Alkylgruppe mit 1 bis 5 Kohlenstoffatomen oder M und N gemeinsam eine Alkylengruppe mit 2 bis 7 Kohlenstoffatomen und n die Zahl 0 bedeutet.

3. Verfahren zur Herstellung der enantiomerenreinen acetalisch mono-geschützten Diole der allgemeinen Formel I gemäss Anspruch 1, dadurch gekennzeichnet, dass man

a) ein enantiomerenreines acetalisch geschütztes 2-Hydroxybutannitril der allgemeinen Formel

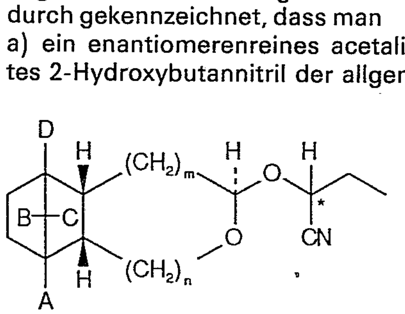

II,

in der A, B, C, D, m und n die in Anspruch 1 angegebene Bedeutung haben, mit einer Grignard-Verbindung der allgemeinen Formel

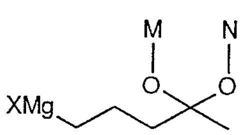

III,

in der X Chlor oder Brom bedeutet, und M und N die in Anspruch 1 angegebenen Bedeutungen haben, zum enantiomerenreinen Keton der allgemeinen Formel

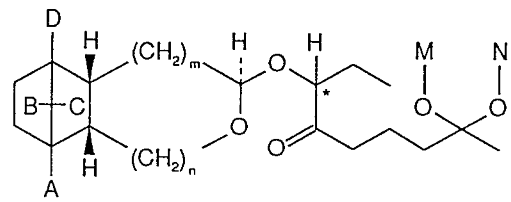

IV,

umsetzt, und

b) dieses Keton zu einem enantiomerenreinen acetalisch mono-geschützten Diastereomeren der allgemeinen Formel

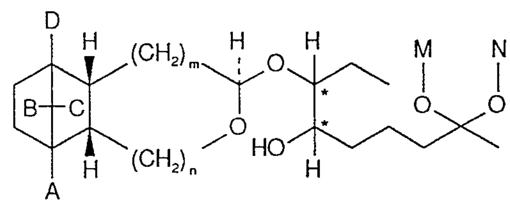

I,

reduziert, wobei je nach den Reaktionsbedingungen die beiden Wasserstoffatome an den mit * gekennzeichneten Asymmetriezentren zueinander bevorzugt entweder in cis-Anordnung oder in trans-Anordnung stehen und gegebenenfalls das als Nebenprodukt gebildete Diastereomere vorzugsweise durch Chromatographie abtrennt.

4. Verfahren gemäss Anspruch 3, dadurch gekennzeichnet, dass man das Keton der allgemeinen Formel IV mit Hilfe eines komplexen Hydrides, wie Zinkborhydrid, Lithiumaluminiumhydrid oder Natrium-bis-(2-methoxyäthoxy)-aluminiumhydrid in Gegenwart eines geeigneten Lösungsmittels, wie Ether oder aromatische Kohlenwasserstoffe, vorzugsweise nicht cyclische Ether, zu einer enantiomerenreinen Verbindung der allgemeinen Formel I reduziert, wobei die beiden Wasserstoffatome an den mit * gekennzeichneten Asymmetriezentren bevorzugt in cis-Anordnung zueinander stehen und gegebenenfalls das als Nebenprodukt gebildete Diastereomere vorzugsweise durch Chromatographie abtrennt.

5. Verfahren gemäss Anspruch 3, dadurch gekennzeichnet, dass man das Keton der allgemeinen Formel IV mit Hilfe eines nicht komplexen Hydrides, wie Boran oder Alanverbindungen, in Gegenwart eines geeigneten Lösungsmittels, wie aromatische oder aliphatische Kohlenwasserstoffe oder Ether, zu einer enantiomerenreinen Verbindung der allgemeinen Formel I reduziert, wobei die beiden Wasserstoffatome an den mit * gekennzeichneten Asymmetriezentren bevorzugt in trans-Anordnung zueinander stehen und gegebenenfalls das als Nebenprodukt gebildete Diastereomere vorzugsweise durch Chromatographie abtrennt.

6. Verwendung der enantiomerenreinen acetalisch mono-geschützten Diole der allgemeinen Formel I gemäss Anspruch 1 zur Herstellung der

enantiomerenreinen Pheromone (1R-exo)-Brevicomin, (1S-exo)-Brevicomin, (1S-endo)-Brevicomin und (1R-endo)-Brevicomin, dadurch gekennzeichnet, dass man enantiomerenreine Diole der allgemeinen Formel I mit katalytischen Mengen starker Säure behandelt, wobei gleichzeitig die tricyclische acetalische Schutzgruppe und die ketalischen Schutzgruppen abgespaltet werden und Ringschluss zu dem entsprechenden enantiomerenreinen Brevicomin erfolgt.

7. Verwendung nach Anspruch 6, dadurch gekennzeichnet, dass die Behandlung mit starker Säure in Gegenwart einer Mercaptosäure erfolgt.

8. Verfahren zur Herstellung der enantiomerenreinen Pheromone (1R-exo)-Brevicomin, (1S-exo)-Brevicomin, (1S-endo)-Brevicomin und (1R-endo)-Brevicomin, dadurch gekennzeichnet, dass man
a) ein enantiomerenreines acetalisch geschütztes 2-Hydroxybutannitril der allgemeinen Formel

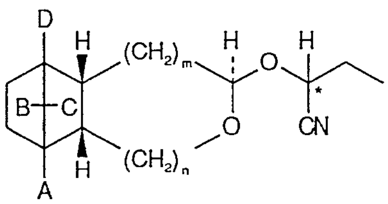
II,

in der A, B, D, C, m und n die in Anspruch 1 angegebene Bedeutung haben, mit einer Grignard-Verbindung der allgemeinen Formel

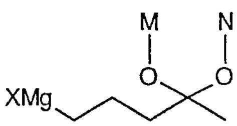
III,

in der X Chlor oder Brom bedeutet, und M und N die in Anspruch 1 angegebene Bedeutung haben, zum enantiomerenreinen Keton der allgemeinen Formel

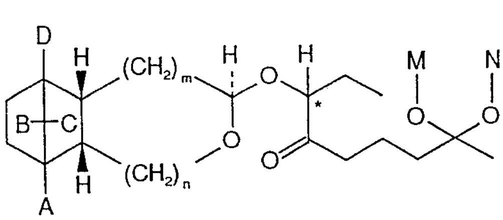
IV,

umsetzt,
b) dieses Keton zu einem enantiomerenreinen acetalisch mono-geschützten Diastereomeren der allgemeinen Formel

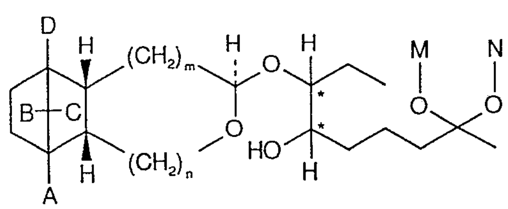
I,

reduziert, wobei je nach Reduktionsmittel die beiden Wasserstoffatome an den mit * gekennzeichneten Asymmetriezentren zueinander bevorzugt entweder in cis-Anordnung oder in trans-Anordnung stehen, gegebenenfalls das als Nebenprodukt gebildete Diastereomere vorzugsweise durch Chromatographie abtrennt und
c) das gebildete neue enantiomerenreine acetalisch mono-geschützte Diol der allgemeinen Formel I mit katalytischen Mengen einer starken Säure behandelt, wobei gleichzeitig die tricyclische acetalische Schutzgruppe und die ketalischen Schutzgruppen abgespaltet werden und Ringschluss zu dem entsprechenden vorgebildeten enantiomerenreinen Pheromon erfolgt.

**Patentansprüche für den Vertragsstaat AT**

1. Verfahren zur Herstellung enantiomerenreiner acetalisch mono-geschützter Diole der allgemeinen Formel

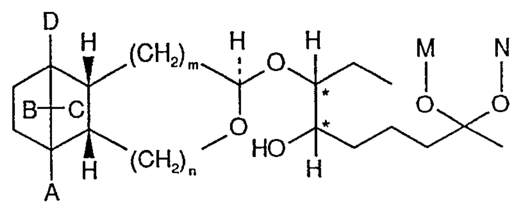
I,

in welcher A, B, C und D Wasserstoff oder eine Methylgruppe in beliebiger Kombination, M und N Keton-Schutzgruppen vom Ketal-Typ, m und n die Zahlen 0, 1 oder 2 bedeuten, wobei die Summe aus m und n die Zahl 1 oder 2 sein muss, dadurch gekennzeichnet, dass man
a) ein enantiomerenreines acetalisch geschütztes 2-Hydroxybutannitril der allgemeinen Formel

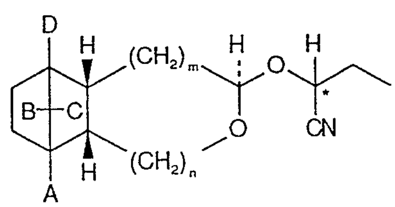
II,

in der A, B, C, D, m und n die obgenannte Bedeutung haben, mit der Grignard-Verbindung der allgemeinen Formel

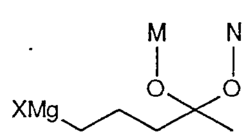
III,

in der X Chlor oder Brom bedeutet und M und N die obgenannte Bedeutung haben, zum enantiomerenreinen Keton der allgemeinen Formel

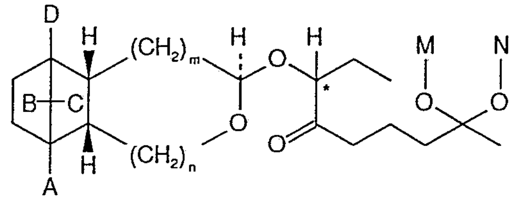
IV,

umsetzt, und

b) dieses Keton zu einem enantiomerenreinen acetalisch mono-geschützten Diastereomeren der allgemeinen Formel I reduziert, wobei je nach den Reaktionsbedingungen die beiden Wasserstoffatome an den mit * gekennzeichneten Asymmetriezentren zueinander bevorzugt entweder in cis-Anordnung oder in trans-Anordnung stehen und gegebenenfalls das als Nebenprodukt gebildete Diastereomere vorzugsweise durch Chromatographie abtrennt.

2. Verfahren gemäss Anspruch 1, dadurch gekennzeichnet, dass man das Keton der allgemeinen Formel IV mit Hilfe eines komplexen Hydrides, wie Zinkborhydrid, Lithiumaluminiumhydrid oder Natrium-bis-(2-methoxyäthoxy)-aluminiumhydrid in Gegenwart eines geeigneten Lösungsmittels, wie Ether oder aromatische Kohlenwasserstoffe, vorzugsweise nicht cyclischer Ether, zu einer enantiomerenreinen Verbindung der allgemeinen Formel I reduziert, wobei die beiden Wasserstoffatome an dem mit * gekennzeichneten Asymmetriezentren bevorzugt in cis-Anordnungen zueinander stehen und gegebenenfalls das als Nebenprodukt gebildete Diastereomere vorzugsweise durch Chromatographie abtrennt.

3. Verfahren gemäss Anspruch 1, dadurch gekennzeichnet, dass man das Keton der allgemeinen Formel IV mit Hilfe eines nicht komplexen Hydrids, wie Boran der Alanverbindungen, in Gegenwart eines geeigneten Lösungsmittels, wie aromatische oder aliphatische Kohlenwasserstoffe oder Ether, zu einer enantiomerenreinen Verbindung der allgemeinen Formel I reduziert, wobei die beiden Wasserstoffatome an den mit * gekennzeichneten Asymmetriezentren bevorzugt in trans-Anordnung zueinander stehen und gegebenenfalls das als Nebenprodukt gebildete Diastereomere vorzugsweise durch Chromatographie abtrennt.

4. Verwendung der enantiomerenreinen acetalisch mono-geschützten Diole der allgemeinen Formel I gemäss Anspruch 1 zur Herstellung der enantiomerenreinen Pheromone (1R-exo)-Brevicomin, (1S-exo)-Brevicomin, (1S-endo)-Brevicomin und (1R-endo)-Brevicomin, dadurch gekennzeichnet, dass man enantiomerenreine Diole der allgemeinen Formel I mit katalytischen Mengen starker Säure behandelt, wobei gleichzeitig die tricyclische acetalische Schutzgruppe und die ketalischen Schutzgruppen abgespalten werden und Ringschluss zu dem entsprechenden enantiomerenreinen Brevicomin erfolgt.

5. Verwendung nach Anspruch 4, dadurch gekennzeichnet, dass die Behandlung mit starker Säure in Gegenwart einer Mercaptosäure erfolgt.

6. Verfahren zur Herstellung der enantiomerenreinen Pheromone (1R-exo)-Brevicomin, (1S-exo)-Brevicomin, (1S-endo)-Brevicomin und (1R-endo)-Brevicomin, dadurch gekennzeichnet, dass man

a) ein enantiomerenreines acetalisch geschütztes 2-Hydroxybutannitril der allgemeinen Formel

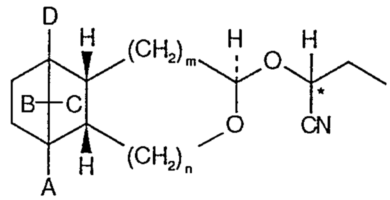
II,

in der A, B, C, D, m und n die in Anspruch 1 angegebene Bedeutung haben, mit einer Grignard-Verbindung der allgemeinen Formel

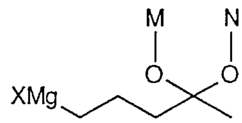
III,

in der X Chlor oder Brom bedeutet, und M und N die in Anspruch 1 angegebene Bedeutung haben, zum enantiomerenreinen Keton der allgemeinen Formel

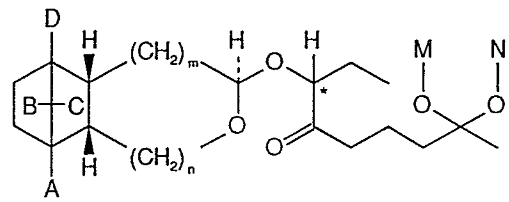
IV,

umsetzt,

b) dieses Keton zu einem enantiomerenreinen acetalisch mono-geschützten Diastereomeren der allgemeinen Formel

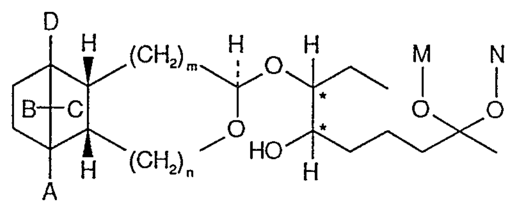
I,

reduziert, wobei je nach den Reaktionsbedingungen die beiden Wasserstoffatome an den mit * gekennzeichneten Asymmetriezentren zueinander bevorzugt entweder in cis-Anordnung oder in trans-Anordnung stehen, gegebenenfalls das als Nebenprodukt gebildete Diastereomere vorzugsweise durch Chromatographie abtrennt und

c) das gebildete neue enantiomerenreine acetalisch mono-geschützte Diol der allgemeinen Formel I mit katalytischen Mengen einer starken Säure behandelt, wobei gleichzeitig die tricyclische acetalische Schutzgruppe und die ketalischen Schutzgruppen abgespalten werden und Ringschluss zu dem entsprechenden vorgebildeten enantiomerenreinen Pheromon erfolgt.

**Claims for the contracting states BE, CH, DE, FR, GB, IT, LI, LU, NL and SE:**

1. Pure enantiomeric diols, mono-protected by an acetal group, of the general formula

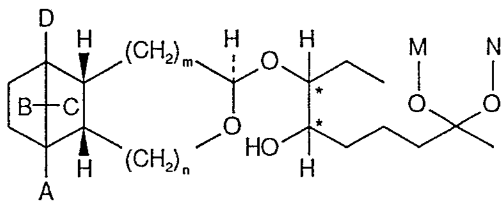

I,

in which A, B, C and D denote hydrogen or a methyl group in any combination, M and N denote ketone protective groups of the ketal type, m and n denote the numbers 0, 1 or 2, where the sum of m and n must be the number 1 or 2.

2. Pure enantiomeric diols, mono-protected by an acetal group, of the general formula I according to Claim 1, characterized in that A, B and C in each case denote a methyl group, D denotes hydrogen, M and N in each case denote an alkyl group having 1 to 5 carbon atoms or M and N together denote an alkylene group having 2 to 7 carbon atoms and n denotes the number 0.

3. Process for the preparation of the pure enantiomeric diols, which are mono-protected by an acetal group, of the general formula I according to Claim 1, characterized in that

a) a pure enantiomeric 2-hydroxybutanenitrile, protected by an acetal group, of the general formula

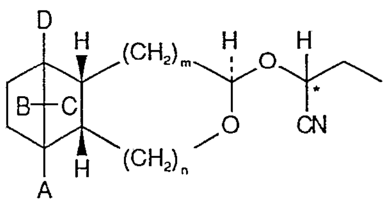

II,

in which A, B, C, D, m and n have the meaning indicated in Claim 1, is reacted with a Grignard compound of the general formula

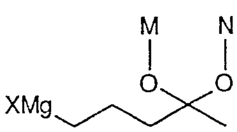

III,

in which X denotes chlorine or bromine and M and N have the meaning indicated in Claim 1, to give a pure enantiomeric ketone of the general formula

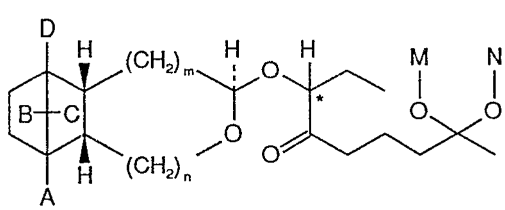

IV,

and

b) this ketone is reduced to a pure enantiomeric diastereomer, mono-protected by an acetal group, of the general formula

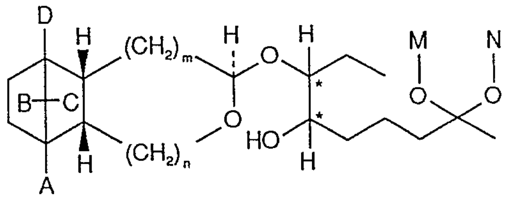

I,

where, depending on the reaction conditions, the two hydrogen atoms on the asymmetric centres identified by * are preferably either in a cis arrangement or a trans arrangement to one another and, if appropriate, the diastereomer formed as by-product is preferably separated off by chromatography.

4. Process according to Claim 3, characterized in that the ketone of the general formula IV is reduced with the aid of a complex hydride, such as zinc borohydride, lithium aluminium hydride or sodium bis-(2-methoxyethoxy) aluminium hydride in the presence of a suitable solvent, such as ethers or aromatic hydrocarbons, preferably noncyclic ethers, to give a pure enantiomeric compound of the general formula I, where the two hydrogen atoms on the asymmetric centres identified by * are preferably in a cis arrangement to one another and, if appropriate, the diastereomer formed as by-product is preferably separated off by chromatography.

5. Process according to Claim 3, characterized in that the ketone of the general formula IV is reduced with the aid of a non-complex hydride, such as borane or alane compounds, in the presence of a suitable solvent, such as aromatic or aliphatic hydrocarbons or ethers, to give a pure enantiomeric compound of the general formula I, where the two hydrogen atoms on the asymmetric centres identified by * are preferably in a trans arrangement to one another and, if appropriate, the diastereomer formed as byproduct is preferably separated off by chromatography.

6. Use of the pure enantiomeric diols, mono-protected by an acetal group, of the general formula I according to Claim 1 for the preparation of the pure enantiomeric pheromones (1R-exo)-brevicomin, (1S-exo)-brevicomin, (1S-endo)-brevicomin and (1R-endo)-brevicomin, characterized in that pure enantiomeric diols of the general formula I are treated with catalytic amounts of strong acid, as a result of which the tricyclic acetal protective group and the ketal protective groups are simultaneously removed and ring closure to the corresponding pure enantiomeric brevicomin takes place.

7. Use according to Claim 6, characterized in that the treatment with strong acid takes place in the presence of a mercapto acid.

8. Process for the preparation of the pure enantiomeric pheromones (1R-exo)-brevicomin, (1S-exo)-brevicomin, (1S-endo)-brevicomin and

(1R-endo)-brevicomin, characterized in that
a) a pure enantiomeric 2-hydroxybutanenitrile, protected by an acetal group, of the general formula

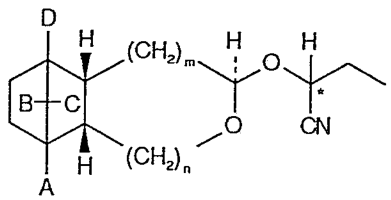

II,

in which A, B, D, C, m and n have the meaning indicated in Claim 1, is reacted with a Grignard compound of the general formula

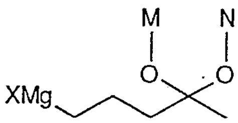

III,

in which X denotes chlorine or bromine, and M and N have the meaning indicated in Claim 1, to give a pure enantiomeric ketone of the general formula

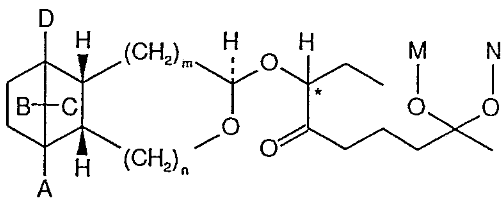

IV,

b) this ketone is reduced to give a pure enantiomeric diastereomer, mono-protected by an acetal group, of the general formula

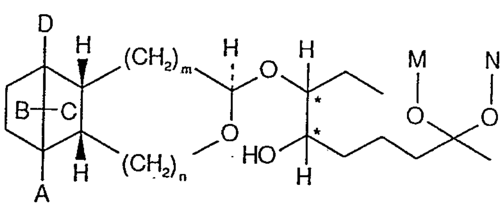

I,

where, depending on the reducing agent, the two hydrogen atoms on the asymmetric centres identified by * are preferably either in a cis arrangement or a trans arrangement to one another and, if appropriate, the diastereomer formed as by-product is preferably separated off by chromatography and
c) the new pure enantiomeric diol, mono-protected by acetal group, of the general formula I formed is treated with catalytic amounts of a strong acid, as a result of which the tricyclic acetal protective group and the ketal protective groups are removed simultaneously and ring closure to the pure enantiomeric pheromone, which has correspondingly been prepared previously takes place.

**Claims for the contracting state AT**

1. Process for the preparation for pure enantiomeric diols, mono-protected by an acetal group, of the general formula

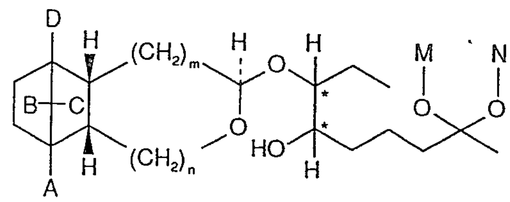

I,

in which A, B, C and D denote hydrogen or a methyl group in any combination, M an N denote ketone protective groups of the ketal type, m and n denote the numbers 0, 1 or 2, where the sum of m and n must be the number 1 or 2, characterized in that
a) a pure enantiomeric 2-hydroxybutanenitrile, protected by an acetal group, of the general formula

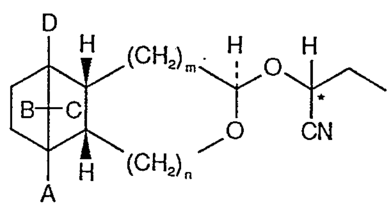

II,

in which A, B, C, D, m and n have the abovementioned meaning, is reacted with the Grignard compound of the general formula

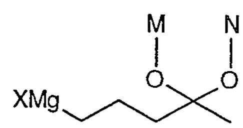

III,

in which X denotes chlorine or bromine and M and N have the abovementioned meaning, to give a pure enantiomeric ketone of the general formula

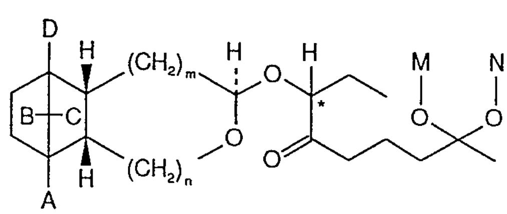

IV,

and
b) this ketone is reduced to a pure enantiomeric diastereomer, mono-protected by an acetal group, of the general formula I where, depending on the reaction conditions, the two hydrogen atoms on the asymmetric centres identified by * are preferably either in a cis arrangement or a trans arrangement to one another and, if appropriate,

the diastereomer formed as by-product is preferably separated off by chromatography.

2. Process according to Claim 1, characterized in that the ketone of the general formula IV is reduced with the aid of a complex hydride, such as zinc borohydride, lithium aluminium hydride or sodium bis-(2-methoxyethoxy) aluminium hydride in the presence of a suitable solvent, such as ethers or aromatic hydrocarbons, preferably noncyclic ethers, to give a pure enantiomeric compound of the general formula I, where the two hydrogen atoms on the asymmetric centres identified by * are preferably in a cis arrangement to one another and, if appropriate, the diastereomer formed as by-product is preferably separated off by chromatography.

3. Process according to Claim 1, characterized in that the ketone of the general formula IV is reduced with the aid of a non-complex hydride, such as borane or alane compounds, in the presence of a suitable solvent, such as aroatic or aliphatic hydrocarbons or ethers, to give a pure enantiomeric compound of the general formula I, where the two hydrogen atoms on the asymmetric centres identified by * are preferably in a trans arrangement to one another and, if appropriate, the disastereomer formed as byproduct is preferably separated off by chromatography.

4. Use of the pure enantiomeric diols, mono-protected by an acetal group, of the general formula I according to Claim 1 for the preparation of the pure enantiomeric pheromones (1R-exo)-brevicomin, (1S-exo)-brevicomin, (1S-endo)-brevicomin and (1R-endo)-brevicomin, characterized in that pure enantiomeric diols of the general formula I are treated with catalytic amounts of strong acid, as a result of which the tricyclic acetal protective group and the ketal protective groups are simultaneously removed and ring closure to the corresponding pure enantiomeric brevicomin takes place.

5. Use according to Claim 4, characterized in that the treatment with strong acid takes place in the presence of a mercapto acid.

6. Process for the preparation of the pure enantiomeric pheromones (1R-exo)-brevicomin, (1S-exo)-brevicomin, (1S-endo)-brevicomin and (1R-endo)-brevicomin, characterized in that

a) a pure enantiomeric 2-hydroxybutanenitrile, protected by an acetal group, of the general formula

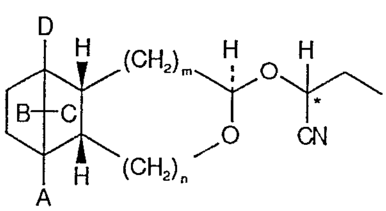

II,

in which X denote chlorine or bromine, and M and N have the meaning indicated in Claim 1, to give a pure enantiomeric ketone of the general formula

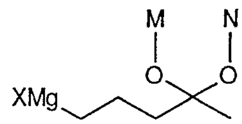

III,

in which X denote chlorine or bromine, and M and N have the meaning indicated in Claim 1, to give a pure enantiomeric ketone of the general formula

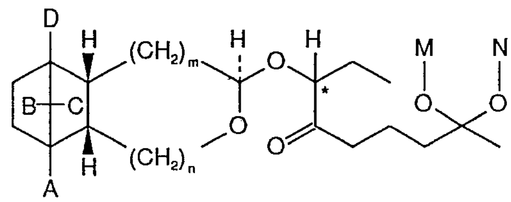

IV,

b) this ketone is reduced to give a pure enantiomeric diastereomer, mono-protected by an acetal group, of the general formula

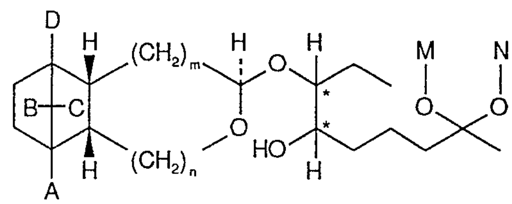

I,

where, depending on the reaction conditions, the two hydrogen atoms on the asymmetric centres identified by * are preferably either in a cis arrangement or a trans arrangement to one another and, if appropriate, the diastereomer formed as by-product is preferably separated off by chromatography and the new pure enantiomeric diol, mono-protected by an acetal group, of the general formula I formed is treated with catalytic amounts of a strong acid, as a result of which the tricyclic acetal protective group and the ketal protective groups are removed simultaneously and ring closure to the pure enantiomeric pheromone, which has correspondingly been prepared previously, takes place.

**Revendications pour les Etats Contractants BE, CH, DE, FR, GB, IT, LI, LU, NL, SE**

1. Diols à mono-protection acétalique de forme énantiomère pure répondant à la formule générale:

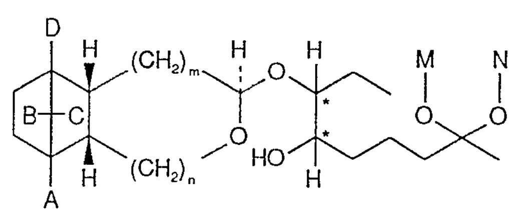

I,

dans laquelle A, B, C et D représentent l'hydrogène ou un groupe méthyle en combinaison quelconque, M et n représentent des groupes cétoniques de type cétalique; m et n représentent les nombres 0, 1 ou 2, la somme de m et n devant être égale à 1 ou 2.

2. Diols à mono-protection acétalique de forme énantiomère pure répondant à la formule générale I selon la revendication 1, caractérisés en ce que A, B et C représentent chacun un groupe méthyle, D représente l'hydrogène, M et N représentent chacun un groupe alkyle comportant 1 à 5 atomes de carbone, ou M et N représentent ensemble un groupe alkylène comportant 2 à 7 atomes de carbone et n représente le nombre 0.

3. Procédé de préparation de diols à mono-protection acétalique de forme énantiomère pure répondant à la formule générale I selon la revendication 1, caractérisé en ce que:

a) on fait réagir un 2-hydroxybutane-nitrile à protection acétalique de forme énantiomère pure répondant à la formule générale:

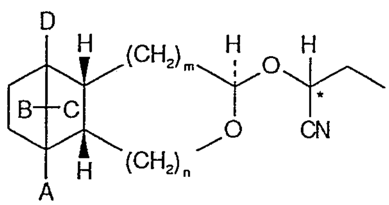

II,

dans laquelle A, B, C, D, m et n ont la signification indiquée dans la revendication 1, sur un composé de Grignard répondant à la formule générale:

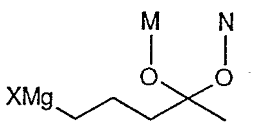

III,

dans laquelle X représente le chlore ou le brome et M et N ont la signification indiquée dans la revendication 1, pour aboutir à la cétone de forme énantiomère pure répondant à la formule générale:

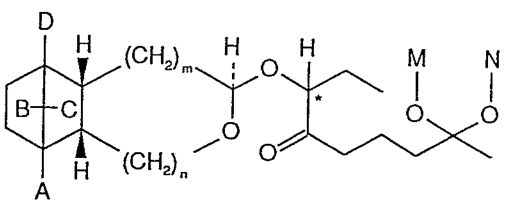

IV,

et,

b) on réduit cette cétone en un diastéréoisomère à mono-protection acétalique de forme énantiomère pure répondant à la formule générale:

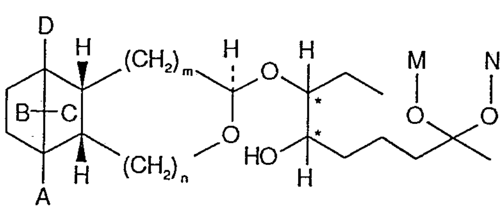

I,

les deux atomes d'hydrogène étant, selon les conditions de réaction, l'un par rapport à l'autre en position cis ou en position trans par rapport aux centres d'asymétrie désignés par *, et le diastéréoisomère formé en tant que produit secondaire étant le cas échéant séparé, de préférence, par chromatographie.

4. Procédé selon la revendication 3, caractérisé en ce qu'on réduit la cétone répondant à la formule générale IV, à l'aide d'un hydrure complexe, comme l'hydrure de bore-zinc, l'hydrure d'aluminium-lithium ou l'hydrure de sodium-bis-(2-méthoxyéthoxy)-aluminium, en présence d'un solvant approprié, comme des éthers ou des carbures d'hydrogène aromatiques, de préférence des éthers non-cycliques, en un composé de forme énantiomère pure répondant à la formule générale I, les deux atomes d'hydrogène étant l'un par rapport à l'autre de préférence en position cis par rapport aux centres de symétrie désignés par * et le diastéréoisomère formé en tant que produit secondaire étant, le cas échéant, séparé de préférence par chromatographie.

5. Procédé selon la revendication 3, caractérisé en ce qu'on réduit la cétone répondant à la formule générale IV, à l'aide d'un hydrure non-complexe, comme le borane ou des composés alaniques, en présence d'un solvant approprié, comme des carbures d'hydrogène aromatiques ou aliphatiques ou bien des éthers, en un composé de forme énantiomère pure répondant à la formule générale I, les deux atomes d'hydrogène étant l'un par rapport à l'autre en position trans par rapport aux centres de symétrie désignés par * et le diastéréoisomère formé en tant que produit secondaire étant, le cas échéant, séparé de préférence par chromatographie.

6. Utilisation des diols à mono-protection acétalique de forme énantiomère pure répondant à la formule générale I selon la revendication 1 pour la préparation des phéromones de forme énantiomère pure (1R-exo)-brévicomine, (1S-exo)-brévicomine, (1S-endo)-brévicomine et (1R-endo)-brévicomine, caractérisée en ce qu'on traite des diols de forme énantiomère pure répondant à la formule générale I par des quantités catalytiques d'acide fort, le groupe de protection acétalique tricyclique et les groupes de protection cétaliques étant en même temps séparés et une cyclisation en la brévicomine de forme énantiomère pure correspondante ayant lieu.

7. Utilisation selon la revendication 6, caractérisée en ce que le traitement par un acide fort a lieu en présence d'un mercapto-acide.

8. Procédé de préparation des phéromones de forme énantiomère pure (1R-exo)-brévicomine, (1S-exo)-brévicomine, (1S-endo)-brévicomine et (1R-endo)-brévicomine, caractérisé en ce que:

a) on fait réagir un 2-hydroxybutane-nitrile à protection acétalique de forme énantiomère pure répondant à la formule générale:

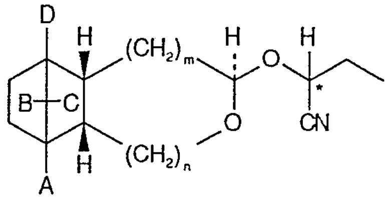

II,

dans laquelle A, B, D, C, m et n ont la signification indiquée dans la revendication 1, sur un composé de Grignard répondant à la formule générale:

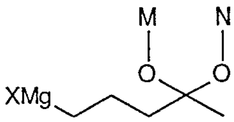

III,

dans laquelle X représente le chlore ou le brome, et M et N ont la signification indiquée dans la revendication 1, pour obtenir une cétone de forme énantiomère pure répondant à la formule générale:

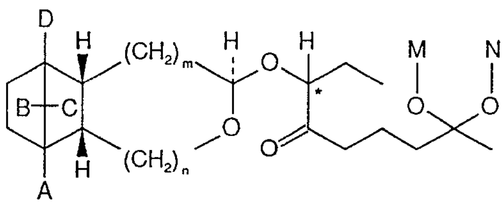

IV,

b) on réduit cette cétone en un diastéréoisomère à mono-protection acétalique de forme énantiomère pure répondant à la formule générale:

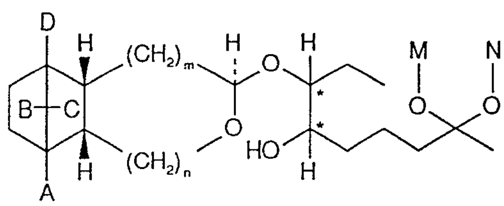

I,

les deux atomes d'hydrogène étant l'un par rapport à l'autre, selon les réducteurs, de préférence en position cis ou en position trans par rapport aux centres d'asymétrie caractérisés par *, et le diastéréoisomère formé en tant que produit secondaire étant séparé le cas échéant de préférence par chromatographie, et

c) on traite le nouveau diol à mono-protection acétalique de forme énantiomère pure formé, répondant à la formule générale I, par des quantités catalytiques d'un acide fort, le groupe de protection acétalique tricyclique et les groupes de protection cétaliques étant en même temps séparés et la cyclisation en la phéromone de forme énantiomère pour préformée correspondante ayant lieu.

**Revendications pour l'Etat Contractant AT**

1. Procédé de préparation de diols à monoprotection acétalique de forme énantiomère pure répondant à la formule générale:

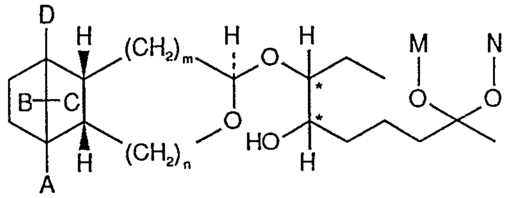

I,

dans laquelle A, B, C et D désignent l'hydrogène ou un groupe méthyle en combinaison quelconque, M et N désignent des groupes de protection cétoniques du type cétal, m et n représentent les nombres 0, 1 ou 2, la somme de m et n devant être égale au nombre 1 ou 2, caractérisé en ce que:

a) on fait réagir un 2-hydroxybutane-nitrile à protection acétalique de forme énantiomère pure répondant à la formule générale:

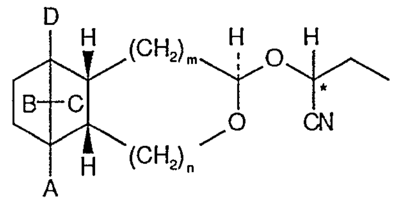

II,

dans laquelle A, B, C, D, m et n ont la signification précitée, sur le composé de Grignard répondant à la formule générale:

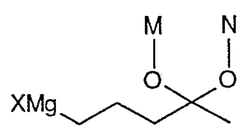

III,

dans laquelle X représente le chlore ou le brome et M et N ont la signification précitée, pour obtenir la cétone de forme énantiomère pure répondant à la formule générale:

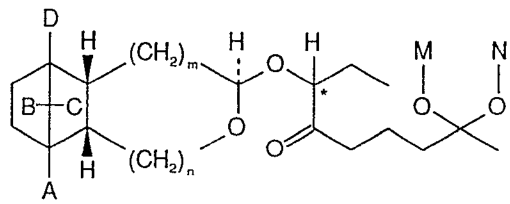

IV,

et

b) on réduit cette cétone en un diastéréoisomère à mono-protection acétalique de forme énantiomère pure répondant à la formule générale I, les deux atomes d'hydrogène étant, selon les conditions de réaction, l'un par rapport à l'autre de préférence en position cis ou en position trans par rapport aux centres d'asymétrie désigné par *, et le diastéréoisomère formé en tant que produit secondaire étant séparé, le cas échéant, de préférence par chromatographie.

2. Procédé selon la revendication 1, caractérisé en ce qu'on réduit la cétone de formule générale IV à l'aide d'un hydrure complexe, tel que

l'hydrure de bore-zinc, l'hydrure d'aliuminium-lithium ou l'hydrure de sodium-bis-(2-méthoxy-éthoxy)-aluminium, en présence d'un solvant approprié, tel que des éthers ou des hydrocarbures aromatiques, de préférence des éthers non-cycliques, en un composé de forme énantiomère pure répondant à la formule générale I, les deux atomes d'hydrogène étant l'un par rapport à l'autre de préférence en position cis par rapport aux centres d'asymétrie caractérisés par *, et le diastéréoisomère formé en tant que produit secondaire étant le cas échéant séparé, de préférence par chromatographie.

3. Procédé selon la revendication 1, caractérisé en ce qu'on réduit la cétone de formule générale IV à l'aide d'un hydrure non complexe tel que le borane ou des composés alaniques, en présence d'un solvant approprié, tel que des hydrocarbures aromatiques ou aliphatiques ou des éthers, en un composé de forme énantiomère pure de formule générale I, les deux atomes d'hydrogène étant l'un par rapport à l'autre, de préférence, en position trans par rapport aux centres d'asymétrie caractérisés par *, et le diastéréoisomère formé en tant que produit secondaire étant le cas échéant séparé, de préférence par chromatographie.

4. Utilisation des diols à mono-protection acétalique de forme éantiomère pure répondant à la formule générale I selon la revendication 1, pour la préparation des phéromones de forme énantiomère pure (1R-exo)-brévicomine, (1S-exo)-brévicomine, (1S-endo)-brévicomine, et (1R-endo)-brévicomine, caractérisée en ce qu'on traite des diols de forme énantiomère pure de formule générale I par des quantités catalytiques d'acide de fort, le groupe de protection acétalique tricyclique et les groupes de protection cétaliques se séparant en même temps et la cyclisation en la brévicomine de forme énantiomère pure correspondante ayant lieu.

5. Utilisation selon la revendication 4, caractérisée en ce que le traitement par l'acide fort a lieu en présence d'un mercapto-acide.

6. Procédé de préparation des phéromones de forme énantiomère pure (1R-exo)-brévicomine, (1S-exo)-brévicomine, (1S-endo)-brévicomine et (1R-endo)-brévicomine, caractérisé en ce que

a) l'on fait réagir un 2-hydroxybutane-nitrile à protection acétalique de forme énantiomère pure répondant à la formule générale:

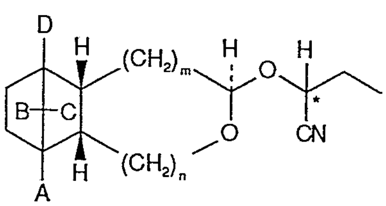

II,

dans laquelle A, B, D, C, m et n ont la signification indiquée dans la revendication 1, sur un composé de Grignard répondant à la formule générale:

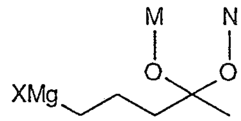

III,

dans laquelle X représente le chlore ou le brome, et M et N ont la signification indiquée dans la revendication 1, pour obtenir la cétone de forme énantiomère pure répondant à la formule générale:

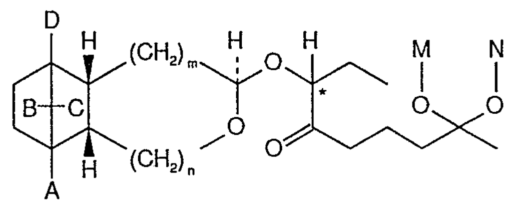

IV,

b) on réduit cette cétone en un diastéréoisomère à mono-protection acétalique de forme énantiomère répondant à la formule générale:

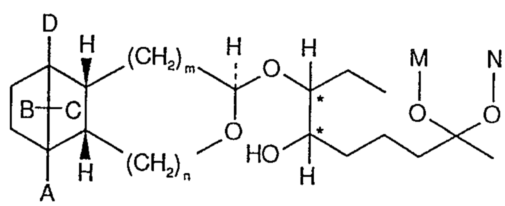

I,

les deux atomes d'hydrogène étant l'un par rapport à l'autre, selon les conditons de réaction, par rapport aux centres d'asymétrie désignés par *, de préférence en position cis ou en position trans, le diastéréoisomère formé en tant que produit secondaire étant le cas échéant séparé, de préférence par chromatographie, et

c) on traite le nouveau diol à mono-protection acétalique de forme énantiomère pur répondant à la formule générale I formé par des quantités catalytiques d'un acide fort, le groupe de protection acétalique tricyclique et les groupes de protection cétalique étant séparé en même temps et la cyclisation en la phéromone de forme énantiomère pure envisagée correspondante ayant lieu.